# EUROPEAN PATENT APPLICATION

(11) **EP 4 817 630 A1**
(43) Date of publication of application: **07.10.2026**
(21) Application number: 24898240.7
(22) Date of filing: 29.11.2024
(51) Int. Cl.: A61K 39/395, A61K 9/08, A61K 47/26, A61K 47/18, A61K 9/00, A61P 17/00, A61P 11/00, A61P 11/02, A61P 29/00, C07K 16/28

(54) **STABLE BUFFER-FREE LIQUID FORMULATION OF ANTI-IL-4R alpha ANTIBODY**

(30) Priority: 01.12.2023 KR 20230172610
(71) Applicant: Samsung Bioepis Co., Ltd., Yeonsu-gu Incheon 21987 (KR)
(72) Inventor: PARK, Jin Hye, Incheon 21982 (KR); KIM, Han Soo, Incheon 22001 (KR); NAM, Myung Joo, Incheon 21982 (KR); KIM, In Ae, Seoul 05090 (KR); LEE, Hun Joo, Incheon 21996 (KR); CHANG, Sung Keun, Incheon 22003 (KR)
(74) Representative: Boult Wade Tennant LLP
(86) International application number: PCT/KR2024/019379
(87) International publication number: WO 2025/116648

(57) **Abstract**

The present disclosure relates to a liquid formulation comprising an anti-IL-4Rα antibody, a saccharide, and an amino acid, free of buffer, and having a pH of 4.5 to 6.5, a device comprising the same, and a use thereof for treating an IL-4Rα-related condition. Despite being free of buffer, the liquid formulation may secure the stability of the antibody while simultaneously improving viscosity, and thus can be usefully utilized as a medicament for treating an IL-4Rα-related condition.

## Description

### Technical Field

The present disclosure relates to a stable buffer-free liquid formulation of an anti-IL-4Rα antibody, a device comprising the same, and a use thereof for treating an IL-4Rα-related condition.

### Background Art

Antibody drugs have a large molecular weight and complex secondary/higher-order structures compared to general protein drugs, which may lead to physicochemical instability. For this reason, the development of an optimal formulation that guarantees the quality and stability of antibody drugs throughout the entire process of manufacture, storage, and administration to a patient is required. Protein instability can arise from various external factors such as temperature, light, and chemicals, which may reduce activity and decrease drug efficacy, or cause immunogenicity upon administration to the human body.

In addition, in the case of high-concentration protein drugs, the viscosity of the solution may increase as the protein concentration increases, due to high inter-protein interactions and crowding. Due to the high-viscosity characteristics of such drugs, the gliding force of a syringe stopper and the injection time may be increased, leading to problems such as reduced patient usability.

Therefore, it is important to address such instability and viscosity of antibody drugs to maintain optimal quality until they are administered to a patient. To achieve optimal quality, methods such as changing the buffer and the stabilizer are used. Because each antibody protein has different material properties, and the optimal combinations of a buffer solution, a pH, a stabilizer, and the like may vary, the development of a formulation suitable for the target material is required.

Meanwhile, in the case of high-concentration protein drugs, a constant pH may be maintained even without including a buffer in some cases, due to a self-buffering effect resulting from the high protein concentration. In addition, some buffers are prone to self-degradation under stress conditions, which may affect the stability of antibody drugs.

Therefore, there is a need to develop a preparation capable of stabilizing antibody protein even without including a buffer that may affect the stability of an anti-IL-4Rα antibody pharmaceutical preparation.

### Disclosure of Invention

### Technical Problem

The present disclosure relates to a stable buffer-free liquid formulation of an anti-IL-4Rα antibody such as dupilumab. Specifically, the present disclosure provides a liquid formulation of an anti-IL-4Rα antibody such as dupilumab, which has high stability and low viscosity even without comprising a buffer.

One aspect is to provide a liquid formulation comprising an anti-IL-4Rα antibody, a saccharide, and an amino acid, and free of buffer.

Another aspect is to provide a device comprising the liquid formulation.

Another aspect is to provide a method of treating an IL-4Rα-related condition, comprising administering the liquid formulation to a subject in need thereof.

Another aspect is to provide a use of the liquid formulation in the manufacture of a medicament for treating an IL-4Rα-related condition.

### Solution to Problem

Unless otherwise defined, all technical terms used herein have the same meaning as commonly understood by a person of ordinary skill in the art to which the present disclosure pertains. In addition, although preferred methods or samples are described herein, those similar or equivalent thereto are also included within the scope of the present disclosure. Furthermore, the numerical values described herein are considered to include the meaning of "about" even when not explicitly stated. The contents of all publications cited as references herein are incorporated herein by reference in their entirety.

As used herein, the term "about" or "approximately" may generally be interpreted to include values or ranges within ±10 %, within ±5 %, within ±4 %, within ±3 %, within ±2 %, or within ±1% of a given value or range.

One aspect provides a stable buffer-free liquid formulation of an anti-IL-4Rα antibody, and specifically, provides a liquid formulation comprising:
an anti-IL-4Rα antibody;
a saccharide; and
an amino acid,
wherein the liquid formulation is free of buffer.

### (1) Antibody

As used herein, the term "antibody" may be interpreted to include a full-length antibody or an antigen-binding fragment thereof. The antibody includes a monoclonal antibody, a polyclonal antibody, a humanized antibody, a human antibody, and a chimeric antibody.

The term "antigen-binding fragment" refers to a fragment including an antigen-binding site of an antibody. For example, the antigen-binding fragment includes, but is not limited to, a Fab fragment, an F(ab')₂ fragment, an Fc fragment, or an scFv fragment.

As used herein, the antibody may be an anti-IL-4Rα antibody. The anti-IL-4Rα antibody may refer to any antibody that binds to the interleukin-4 receptor alpha chain (IL-4Rα). The anti-IL-4Rα antibody can inhibit IL-4 and IL-13 signaling.

The anti-IL-4Rα antibody may be dupilumab (CAS No. 1190264-60-8). Therefore, the liquid formulation may be a stable liquid formulation of dupilumab. Dupilumab is a fully human monoclonal antibody that binds to IL-4Rα, and it belongs to IgG4. Dupilumab is sold under the trade name Dupixent^{®}. After being approved by the US Food and Drug Administration (FDA) as a treatment for atopic dermatitis, Dupixent is expanding its indications to various type 2 inflammatory diseases such as asthma, chronic rhinosinusitis with nasal polyposis (CRSwNP), and eosinophilic esophagitis (EoE). The sequence of dupilumab is known, and dupilumab may be produced by general methods known in the art. More detailed information on dupilumab may be easily obtained from known databases by a person skilled in the art.

As used herein, the term "dupilumab" may also be construed to encompass dupilumab in which the amino acid sequence is modified (by deletion, insertion, and/or substitution) and/or the glycosylation profile is modified, to an extent that it does not alter the polypeptide function.

The anti-IL-4Rα antibody may be included in the liquid formulation in a therapeutically effective amount.

The concentration of the anti-IL-4Rα antibody may be about 1 mg/mL to about 300 mg/mL, about 1 mg/mL to about 250 mg/mL, about 1 mg/mL to about 200 mg/mL, about 1 mg/mL to about 175 mg/mL, about 5 mg/mL to about 300 mg/mL, about 5 mg/mL to about 250 mg/mL, about 5 mg/mL to about 200 mg/mL, about 5 mg/mL to about 175 mg/mL, about 10 mg/mL to about 300 mg/mL, about 10 mg/mL to about 250 mg/mL, about 10 mg/mL to about 200 mg/mL, about 10 mg/mL to about 175 mg/mL, about 25 mg/mL to about 300 mg/mL, about 25 mg/mL to about 250 mg/mL, about 25 mg/mL to about 200 mg/mL, about 25 mg/mL to about 175 mg/mL, about 50 mg/mL to about 300 mg/mL, about 50 mg/mL to about 250 mg/mL, about 50 mg/mL to about 200 mg/mL, about 50 mg/mL to about 175 mg/mL, about 75 mg/mL to about 300 mg/mL, about 75 mg/mL to about 250 mg/mL, about 75 mg/mL to about 200 mg/mL, about 75 mg/mL to about 175 mg/mL, about 100 mg/mL to about 300 mg/mL, about 100 mg/mL to about 250 mg/mL, about 100 mg/mL to about 200 mg/mL, about 100 mg/mL to about 175 mg/mL, about 125 mg/mL to about 300 mg/mL, about 125 mg/mL to about 250 mg/mL, about 125 mg/mL to about 200 mg/mL, about 125 mg/mL to about 175 mg/mL, about 145 mg/mL to about 300 mg/mL, about 145 mg/mL to about 250 mg/mL, about 145 mg/mL to about 200 mg/mL, about 145 mg/mL to about 180 mg/mL, about 145 mg/mL to about 175 mg/mL, about 149 mg/mL to about 151 mg/mL, about 150 mg/mL to about 300 mg/mL, about 150 mg/mL to about 250 mg/mL, about 150 mg/mL to about 225 mg/mL, about 150 mg/mL to about 200 mg/mL, about 150 mg/mL to about 180 mg/mL, about 150 mg/mL to about 175 mg/mL, about 170 mg/mL to about 300 mg/mL, about 170 mg/mL to about 250 mg/mL, about 170 mg/mL to about 225 mg/mL, about 170 mg/mL to about 200 mg/mL, about 170 mg/mL to about 180 mg/mL, or about 174 mg/mL to about 176 mg/mL.

In an embodiment, the concentration of the anti-IL-4Rα antibody may be about 100 mg/mL to about 200 mg/mL.

In an embodiment, the concentration of the anti-IL-4Rα antibody may be about 125 mg/mL to about 200 mg/mL.

In an embodiment, the concentration of the anti-IL-4Rα antibody may be about 150 mg/mL to about 200 mg/mL.

In an embodiment, the concentration of the anti-IL-4Rα antibody may be about 150 mg/mL to about 175 mg/mL.

In an embodiment, the concentration of the anti-IL-4Rα antibody may be 135 mg/mL to 165 mg/mL.

In an embodiment, the concentration of the anti-IL-4Rα antibody may be 158 mg/mL to 192 mg/mL.

In a specific embodiment, the concentration of the anti-IL-4Rα antibody may be about 150 mg/mL.

In a specific embodiment, the concentration of the anti-IL-4Rα antibody may be about 175 mg/mL.

The concentration of the anti-IL-4Rα antibody may be a high concentration. Therefore, the liquid formulation may be a high-concentration liquid formulation of the anti-IL-4Rα antibody. The high concentration may mean 100 mg/mL or more or 150 mg/mL or more, for example, 100 mg/mL to 300 mg/mL or 150 mg/mL to 300 mg/mL, but is not limited thereto.

### (2) Buffer and pH

The liquid formulation according to one aspect is free of buffer. Therefore, the liquid formulation may be a buffer-free formulation or a bufferless formulation.

The liquid formulation may have high stability and low viscosity despite being a buffer-free formulation.

The term "free of component A" or "substantially free of component A" may be interpreted to include a case where component A is not present at all, a case where, even when component A is present, it is present in a trace amount that does not substantially affect the properties of the formulation, or a case where it is present in an undetectable amount.

As used herein, the phrase "free of buffer" may be interpreted to mean that a buffer component is not present in the formulation, or that a buffer is included in an amount incapable of exhibiting an intended function as a stabilizer buffer in the formulation.

The liquid formulation is free of, or substantially free of, buffer.

The buffer may comprise one or more selected from acetate, histidine, phosphate, citrate, succinate, malate, tartrate, carbonate, salts thereof, and hydrates thereof.

The term "salt" may refer to a pharmaceutically acceptable salt. The salt may comprise an inorganic acid salt, an organic acid salt, a metal salt, and the like of the compound. The inorganic acid salt may be a hydrochloride, hydrobromide, phosphate, sulfate, or disulfate. The organic acid salt may be a formate, acetate, propionate, lactate, oxalate, tartrate, malate, maleate, citrate, fumarate, besylate, camsylate, edisylate, trichloroacetate, trifluoroacetate, benzoate, gluconate, methanesulfonate, glycolate, succinate, 4-toluenesulfonate, galacturonate, embonate, glutamate, ethanesulfonate, benzenesulfonate, p-toluenesulfonate, or aspartate. The metal salt may be a calcium salt, a sodium salt, a magnesium salt, a strontium salt, or a potassium salt.

The term "hydrate" refers to a substance containing a water molecule within its molecule. The hydrate may be a monohydrate, a dihydrate, or a trihydrate.

In an embodiment, the buffer may comprise acetate. Therefore, the liquid formulation may not comprise acetate as a buffer.

In an embodiment, the buffer may comprise a combination of acetate and histidine. Therefore, the liquid formulation may not comprise either acetate or histidine as a buffer.

The pH of the liquid formulation according to one aspect may be any range or any value selected within about 4.0 to about 8.5. For example, the pH may be about 4.0 to about 8.5, about 4.0 to about 8.0, about 4.0 to about 7.5, about 4.0 to about 7.0, about 4.0 to about 6.5, about 4.0 to about 6.0, about 4.5 to about 8.5, about 4.5 to about 8.0, about 4.5 to about 7.5, about 4.5 to about 7.0, about 4.5 to about 6.5, about 4.5 to about 6.0, about 4.7 to about 8.5, about 4.7 to about 8.0, about 4.7 to about 7.5, about 4.7 to about 7.4, about 4.7 to about 7.0, about 4.7 to about 6.5, about 4.7 to about 6.0, about 5.0 to about 8.5, about 5.0 to about 8.0, about 5.0 to about 7.5, about 5.0 to about 7.0, about 5.0 to about 6.5, about 5.0 to about 6.0, about 5.5 to about 8.5, about 5.5 to about 8.0, about 5.5 to about 7.5, about 5.5 to about 7.0, about 5.5 to about 6.5, about 5.5 to about 6.0, about 5.6 to about 8.5, about 5.6 to about 8.0, about 5.6 to about 7.5, about 5.6 to about 7.0, about 5.6 to about 6.5, about 5.6 to about 6.0, about 5.8 to about 8.5, about 5.8 to about 8.0, about 5.8 to about 7.5, about 5.8 to about 7.0, about 5.8 to about 6.5, about 5.8 to about 6.2, about 5.8 to about 6.1, about 5.8 to about 6.0, about 5.9 to about 8.5, about 5.9 to about 8.0, about 5.9 to about 7.5, about 5.9 to about 7.0, about 5.9 to about 6.5, about 5.9 to about 6.2, about 5.9 to about 6.1, or about 5.9 to about 6.0.

In an embodiment, the pH of the liquid formulation may be about 4.7 to about 7.4.

In a specific embodiment, the pH of the liquid formulation may be about 5.9.

In a specific embodiment, the pH of the liquid formulation may be about 6.0.

### (3) Saccharide

The liquid formulation according to one aspect comprises a saccharide.

The liquid formulation according to one aspect may comprise a saccharide as a stabilizer.

The saccharide may comprise one or more selected from a sugar and a sugar alcohol.

The sugar may be a monosaccharide, a disaccharide, an oligosaccharide, or a polysaccharide. The sugar may comprise one or more selected from sucrose, trehalose, galactose, mannose, maltose, lactose, fructose, and glucose.

The sugar alcohol, which is obtained by reducing an aldehyde group or a ketone group of a sugar to an alcohol group, is a generic term for a polyol having two or more hydroxy groups. The sugar alcohol may comprise one or more selected from mannitol, sorbitol, xylitol, arabitol, erythritol, lactitol, maltitol, and inositol. The sugar alcohol comprises an anhydrous form or a hydrate of the sugar alcohol. For example, trehalose may comprise not only trehalose but also trehalose dihydrate.

The concentration of the sugar may be freely adjusted within a range that maintains the stability of the antibody, and may vary individually depending on each specific sugar type. The concentration of the sugar may be any range or any value selected within about 0.1% (w/v) to about 20% (w/v). For example, the concentration of the sugar may be about 0.1% (w/v) to about 20% (w/v), about 0.1% (w/v) to about 15% (w/v), about 0.1% (w/v) to about 12% (w/v), about 0.1% (w/v) to about 10% (w/v), about 0.1% (w/v) to about 8% (w/v), about 0.1% (w/v) to about 6% (w/v), about 1% (w/v) to about 20% (w/v), about 1% (w/v) to about 15% (w/v), about 1% (w/v) to about 12% (w/v), about 1% (w/v) to about 10% (w/v), about 1% (w/v) to about 8% (w/v), about 1% (w/v) to about 6% (w/v), about 2% (w/v) to about 20% (w/v), about 2% (w/v) to about 15% (w/v), about 2% (w/v) to about 12% (w/v), about 2% (w/v) to about 10% (w/v), about 2% (w/v) to about 8% (w/v), about 2% (w/v) to about 6% (w/v), about 3% (w/v) to about 20% (w/v), about 3% (w/v) to about 15% (w/v), about 3% (w/v) to about 12% (w/v), about 3% (w/v) to about 10% (w/v), about 3% (w/v) to about 8% (w/v), about 3% (w/v) to about 6% (w/v), about 4% (w/v) to about 20% (w/v), about 4% (w/v) to about 15% (w/v), about 4% (w/v) to about 12% (w/v), about 4% (w/v) to about 10% (w/v), about 4% (w/v) to about 8% (w/v), or about 4% (w/v) to about 6% (w/v).

The concentration of the sugar alcohol may be freely adjusted within a range that maintains the stability of the antibody, and may vary individually depending on each specific sugar alcohol type. The concentration of the sugar alcohol may be any range or any value selected within about 0.1% (w/v) to about 20% (w/v). For example, the concentration of the sugar alcohol may be about 0.1% (w/v) to about 20% (w/v), about 0.1% (w/v) to about 15% (w/v), about 0.1% (w/v) to about 12% (w/v), about 0.1% (w/v) to about 10% (w/v), about 0.1% (w/v) to about 8% (w/v), about 0.1% (w/v) to about 5% (w/v), about 1% (w/v) to about 20% (w/v), about 1% (w/v) to about 15% (w/v), about 1% (w/v) to about 12% (w/v), about 1% (w/v) to about 10% (w/v), about 1% (w/v) to about 8% (w/v), about 1% (w/v) to about 7% (w/v), about 1% (w/v) to about 6% (w/v), about 1% (w/v) to about 5% (w/v), about 1% (w/v) to about 4% (w/v), about 1% (w/v) to about 3% (w/v), or about 1% (w/v) to about 2% (w/v).

In a specific embodiment, the saccharide may comprise sucrose. In a specific embodiment, the saccharide may be sucrose.

The concentration of the sucrose may be any range or any value selected within about 0.1% (w/v) to about 20% (w/v). For example, the concentration of the sucrose may be about 0.1% (w/v) to about 20% (w/v), about 0.1% (w/v) to about 15% (w/v), about 0.1% (w/v) to about 12% (w/v), about 0.1% (w/v) to about 10% (w/v), about 0.1% (w/v) to about 8% (w/v), about 0.1% (w/v) to about 6% (w/v), about 1% (w/v) to about 20% (w/v), about 1% (w/v) to about 15% (w/v), about 1% (w/v) to about 12% (w/v), about 1% (w/v) to about 10% (w/v), about 1% (w/v) to about 8% (w/v), about 1% (w/v) to about 6% (w/v), about 2% (w/v) to about 20% (w/v), about 2% (w/v) to about 15% (w/v), about 2% (w/v) to about 12% (w/v), about 2% (w/v) to about 10% (w/v), about 2% (w/v) to about 8% (w/v), about 2% (w/v) to about 6% (w/v), about 3% (w/v) to about 20% (w/v), about 3% (w/v) to about 15% (w/v), about 3% (w/v) to about 12% (w/v), about 3% (w/v) to about 10% (w/v), about 3% (w/v) to about 8% (w/v), about 3% (w/v) to about 6% (w/v), about 4% (w/v) to about 20% (w/v), about 4% (w/v) to about 15% (w/v), about 4% (w/v) to about 12% (w/v), about 4% (w/v) to about 10% (w/v), about 4% (w/v) to about 8% (w/v), or about 4% (w/v) to about 6% (w/v).

In a specific embodiment, the concentration of the sucrose may be about 5% (w/v).

### (4) Amino Acid

The liquid formulation according to one aspect comprises an amino acid.

The liquid formulation according to one aspect may comprise an amino acid as a stabilizer.

The term "stabilizer" refers to a substance added to prevent a state change or a chemical change when preserving a substance. The stabilizer may inhibit aggregation or denaturation of the antibody.

The amino acid may comprise one or more selected from glycine, alanine, valine, leucine, isoleucine, proline, phenylalanine, tyrosine, tryptophan, serine, threonine, cysteine, methionine, asparagine, glutamine, lysine, arginine, histidine, aspartic acid, and glutamic acid.

In an embodiment, the amino acid may comprise arginine.

The concentration of the amino acid may be freely adjusted within a range that maintains the stability of the antibody, and may vary individually depending on each specific amino acid type. The concentration of the amino acid may be any range or any value selected within about 1 mM to about 400 mM. For example, the concentration of the amino acid may be about 1 mM to about 400 mM, about 1 mM to about 300 mM, about 1 mM to about 200 mM, about 1 mM to about 100 mM, about 10 mM to about 400 mM, about 10 mM to about 300 mM, about 10 mM to about 200 mM, about 10 mM to about 100 mM, about 10 mM to about 80 mM, about 10 mM to about 60 mM, about 20 mM to about 400 mM, about 20 mM to about 300 mM, about 20 mM to about 200 mM, about 20 mM to about 100 mM, about 20 mM to about 80 mM, about 20 mM to about 60 mM, about 30 mM to about 400 mM, about 30 mM to about 300 mM, about 30 mM to about 200 mM, about 30 mM to about 100 mM, about 30 mM to about 80 mM, about 30 mM to about 60 mM, about 40 mM to about 400 mM, about 40 mM to about 300 mM, about 40 mM to about 200 mM, about 40 mM to about 100 mM, about 40 mM to about 80 mM, or about 40 mM to about 60 mM.

### (5) Surfactant

The liquid formulation according to one aspect may further comprise a surfactant.

Therefore, the liquid formulation may be a liquid formulation comprising an anti-IL-4Rα antibody, a saccharide, an amino acid, and a surfactant, and free of buffer.

The surfactant may be selected from any pharmaceutically acceptable surfactants capable of evenly dispersing a protein (e.g., an antibody) in a liquid formulation medium.

The surfactant may be a nonionic surfactant.

Specifically, the surfactant may be one or more selected from the group consisting of a polysorbate, a poloxamer, a sorbitan ester of another fatty acid, a polyethylene-polypropylene glycol, a polyoxyethylene compound, sodium dodecyl sulphate (SDS), and the like.

The polysorbate may comprise polysorbate 20, polysorbate 40, polysorbate 60, polysorbate 65, polysorbate 80, and polysorbate 85, and the like.

The poloxamer may comprise a PEO-PPO-PEO copolymer (PEO is poly(ethylene oxide) and PPO is poly(propylene oxide)), and the like.

The sorbitan ester of another fatty acid may refer to a sorbitan ester of a fatty acid other than a polysorbate, and may comprise, for example, a sorbitan polyethoxylate, and the like.

The polyoxyethylene compound may comprise polyoxyethylene stearate, a polyoxyethylene alkyl ether (alkyl: C1-C30), polyoxyethylene monolauryl ether, an alkylphenyl polyoxyethylene copolymer (alkyl: C1-C30), and the like.

In an embodiment, the surfactant may be a polysorbate.

In an embodiment, the surfactant may comprise one or more selected from polysorbate 20, polysorbate 40, polysorbate 60, polysorbate 65, polysorbate 80, and polysorbate 85.

In an embodiment, the surfactant may comprise polysorbate 20, polysorbate 80, or a combination thereof.

In a specific embodiment, the surfactant may comprise polysorbate 80. In a specific embodiment, the surfactant may be polysorbate 80.

The concentration of the surfactant may be any range or any value selected within about 0.01% (w/v) to about 0.9% (w/v). For example, the concentration of the surfactant may be about 0.01% (w/v) to about 0.9% (w/v), about 0.01% (w/v) to about 0.5% (w/v), about 0.1% (w/v) to about 0.9% (w/v), about 0.1% (w/v) to about 0.5% (w/v), about 0.1% (w/v) to about 0.4% (w/v), about 0.1% (w/v) to about 0.3% (w/v), about 0.15% (w/v) to about 0.9% (w/v), about 0.15% (w/v) to about 0.5% (w/v), about 0.15% (w/v) to about 0.4% (w/v), about 0.15% (w/v) to about 0.3% (w/v), or about 0.15% (w/v) to about 0.25% (w/v).

In a specific embodiment, the concentration of the surfactant may be about 0.2% (w/v).

### (6) Diluent

The liquid formulation according to one aspect may further comprise a diluent.

The diluent may be an aqueous carrier. The aqueous carrier is a pharmaceutically acceptable carrier that is safe and non-toxic when it is administered to a human, and may be, for example, water, a saline solution, a Ringer's solution, dextrose, or a mixture thereof.

In an embodiment, the diluent may be water. Therefore, the liquid formulation may be an aqueous liquid formulation.

### (7) Formulation

The term "liquid formulation" refers to a formulation in a liquid state.

The liquid formulation according to one aspect is a stable liquid formulation of an anti-IL-4Rα antibody.

The liquid formulation according to one aspect may be a pharmaceutical formulation of an anti-IL-4Rα antibody.

The term "pharmaceutical composition" or "pharmaceutical preparation" refers to a preparation that allows the biological activity of an active ingredient to act effectively, and does not include an additional ingredient having severe toxicity to a subject to which the preparation is to be administered.

The term "pharmaceutical formulation" refers to a product of a process of producing a final drug product by combining an active drug with a chemical substance.

The term "pharmaceutically acceptable" may refer to excipients, carriers, vehicles, diluents, additives, salts, and the like suitable for administration to a subject.

The liquid formulation according to one aspect may be a Dupixent^{®} biosimilar.

The term "biosimilar", also called a "biogeneric", refers to a generic drug for an original biopharmaceutical. Because a biopharmaceutical is not synthesized as a chemical product but is produced through cells, a product perfectly identical to the original drug cannot be replicated. Therefore, a generic drug for a biopharmaceutical is called a biosimilar in the sense that it is similar to, although not identical to, the original drug.

The liquid formulation according to one aspect may be selected from the following items:
1) a liquid formulation comprising an anti-IL-4Rα antibody, a saccharide, and an amino acid, and free of buffer;
2) a liquid formulation comprising about 5 mg/mL to about 300 mg/mL of an anti-IL-4Rα antibody, a saccharide, and an amino acid, and free of buffer;
3) a liquid formulation comprising an anti-IL-4Rα antibody, a saccharide, and an amino acid, and not comprising acetate as a buffer;
4) a liquid formulation comprising an anti-IL-4Rα antibody, a saccharide, and an amino acid, and not comprising either acetate or histidine as a buffer;
5) a liquid formulation comprising an anti-IL-4Rα antibody, a saccharide, an amino acid, and a surfactant, and free of buffer;
6) a liquid formulation comprising about 5 mg/mL to about 300 mg/mL of an anti-IL-4Rα antibody, a saccharide, an amino acid, and a surfactant, and free of buffer;
7) a liquid formulation comprising an anti-IL-4Rα antibody, a saccharide, an amino acid, and a surfactant, and not comprising acetate as a buffer;
8) a liquid formulation comprising an anti-IL-4Rα antibody, a saccharide, an amino acid, and a surfactant, and not comprising either acetate or histidine as a buffer;
9) a liquid formulation comprising dupilumab, sucrose, arginine, and polysorbate 80, and free of buffer; or
10) a liquid formulation comprising about 100 mg/mL to about 200 mg/mL of dupilumab, 2% (w/v) to 10% (w/v) of sucrose, 20 mM to 100 mM of arginine, and 0.1% (w/v) to 0.3% (w/v) of polysorbate 80, free of buffer, and having a pH of about 5.6 to about 6.5.

### (8) Stability and Viscosity

Despite being free of buffer, the liquid formulation according to one aspect may have improved stability and improved viscosity compared to a buffer-containing anti-IL-4Rα antibody liquid formulation.

The term "stability" means that the antibody (e.g., dupilumab) included in the formulation substantially retains its physical stability, chemical stability, and/or biological activity before and after administration, during an additional manufacturing process, or upon preservation or storage. For example, it may be understood to mean that the degree of loss of stability, such as aggregation, degradation, denaturation (acidic or basic), oxidation, and the like of the antibody included in the formulation, is 20 % or less, 15 % or less, 10 % or less, or 5 % or less compared to the initial stage of storage. Therefore, "excellent stability" or "improved stability" may mean a low protein aggregation rate, a low protein degradation rate, a low protein denaturation rate, a low amino acid oxidation rate, and the like during storage. Physical stability, chemical stability, and/or biological activity may be evaluated by commonly known methods.

The term "aggregate" may refer to a high molecular weight (HMW) species formed by aggregation of an antibody protein. The term "protein aggregation rate" may be expressed as a high molecular weight species content (%HMW) of the antibody in the formulation at a certain time point. The %HMW may be measured by size exclusion chromatography (SEC), but is not limited thereto. For example, "improved stability" may mean that the %HMW of the antibody measured for the formulation is decreased by 0.01 % or more, 0.1 % or more, 0.5 % or more, 1 % or more, 2 % or more, 3 % or more, 4 % or more, 5 % or more, 10 % or more, 15 % or more, or 20 % or more compared to an existing formulation. A decrease in the %HMW may mean that the stability is improved because the degree to which aggregation of the antibody in the formulation occurs is decreased.

The stability evaluation may be performed immediately after preparation of the formulation, or after storage for a certain period of time under accelerated stability conditions or stress stability conditions. The accelerated stability conditions may include conditions used in an accelerated test for pharmaceuticals, and may be, for example, a temperature of 25±2 °C and a relative humidity (RH) of 60±5 %. The stress stability conditions may include conditions used in a stress test for pharmaceuticals, and may be, for example, a temperature of 40±2 °C and a relative humidity (RH) of 75+5 %.

The liquid formulation may have a decreased high molecular weight species content (%HMW).

By being free of buffer, the liquid formulation may have a decreased %HMW compared to a buffer-containing anti-IL-4Rα antibody liquid formulation.

In the liquid formulation, the %HMW of the antibody measured after storage at 25 °C for 4 weeks may be decreased by 1 % or more, 2 % or more, or 3 % or more compared to a buffer-containing formulation.

In the liquid formulation, the %HMW of the antibody measured after storage at 25 °C for 4 weeks may be 3.15 % or less, 3.14 % or less, 3.13 % or less, 3.12 % or less, 3.11 % or less, or 3.10 % or less.

The liquid formulation may have an improved viscosity. The 'improvement' of the viscosity may mean a 'decrease' in the viscosity. Therefore, the liquid formulation may have a decreased viscosity.

By being free of buffer, the liquid formulation may have an improved viscosity compared to a buffer-containing anti-IL-4Rα antibody liquid formulation.

The liquid formulation may have a viscosity decreased by 1 % or more, 5 % or more, 10 % or more, 15 % or more, 20 % or more, or 25 % or more compared to the viscosity of a buffer-containing formulation.

The viscosity of the liquid formulation may be less than 11.5 cP (centipoise). The viscosity of the liquid formulation may be less than 11.5 cP, 11.4 cP or less, 11.2 cP or less, 11.0 cP or less, or 10.5 cP or less.

### (9) Device

Another aspect provides a device comprising the liquid formulation according to one aspect.

The device is mainly used for a purpose of parenteral administration. The parenteral administration may include, for example, subcutaneous, intramuscular, intravenous, intraperitoneal, intracerebrospinal, intra-articular, intrasynovial, or intrathecal administration. The device may be accompanied by instructions for administration.

The device may comprise the liquid formulation in a container selected from a syringe, a pre-filled syringe, a pre-filled pen, a microinfuser, an autoinjector, a bottle, a vial, and a tube, but is not limited thereto.

The device may be in a single-dose form or a multiple-dose form.

In an embodiment, the device may comprise the liquid formulation in a pre-filled syringe. The pre-filled syringe may be a single-dose pre-filled syringe.

In an embodiment, the device may comprise the liquid formulation in a pre-filled pen. The pre-filled pen may be a single-dose pre-filled pen.

### (10) Treatment of Disease

Another aspect provides a method of treating an IL-4Rα-related condition, the method comprising administering the liquid formulation according to one aspect to a subject in need thereof. The liquid formulation may be in a form included in a device.

Another aspect provides a use of the liquid formulation according to one aspect in the manufacture of a medicament for treating an IL-4Rα-related condition.

The method of treating an IL-4Rα-related condition may further comprise identifying a subject in need of administration of an anti-IL-4Rα antibody (e.g., dupilumab) prior to the administration.

The subject may be a subject in need of administration of a liquid formulation containing the anti-IL-4Rα antibody (e.g., dupilumab). The subject in need of administration of the liquid formulation containing the anti-IL-4Rα antibody (e.g., dupilumab) may be a subject having a disease or disorder that may be significantly treated (e.g., elimination, mitigation, alleviation, or improvement of symptoms, and the like) by administration of the anti-IL-4Rα antibody. The subject may be selected from mammals including humans.

The liquid formulation may be administered in a pharmaceutically effective amount.

The IL-4Rα-related condition may include any condition, disease, or disorder that may be treated by administration of the anti-IL-4Rα antibody. The IL-4Rα-related condition that may be treated by administration of an anti-IL-4Rα antibody (e.g., dupilumab) may include all indications previously approved or all indications that may be approved in the future for the anti-IL-4Rα antibody (e.g., dupilumab).

The IL-4Rα-related condition may be an IL-4-mediated disease and/or an IL-13-mediated disease. The IL-4-mediated disease may include any disease that may be treated by inhibition of IL-4 signaling. The IL-13-mediated disease may include any disease that may be treated by inhibition of IL-13 signaling. The IL-4Rα-related condition may include any disease that may be treated by a dual blockade of IL-4 and IL-13.

The IL-4Rα-related condition may include an inflammatory disease, an allergic disease, or an autoimmune disease. The inflammatory disease may be a Type 2 inflammatory disease.

The IL-4Rα-related condition may be any one selected from atopic dermatitis, asthma, chronic rhinosinusitis (CRS), chronic rhinosinusitis with nasal polyposis (CRSwNP), allergic fungal rhinosinusitis (AFRS), eosinophilic esophagitis (EoE), prurigo nodularis (PN), bullous pemphigoid (BP), chronic spontaneous urticaria (CSU), chronic inducible urticaria (CIndU), and chronic obstructive pulmonary disease (COPD), but is not limited thereto.

The atopic dermatitis may be moderate-to-severe atopic dermatitis.

The asthma may be moderate-to-severe asthma.

### (11) Route of Administration and Dosage

The liquid formulation according to one aspect may be administered by a parenteral route. The parenteral route may include subcutaneous administration, intravenous administration, and the like. The parenteral administration may be by a bolus injection or a continuous infusion.

In a specific embodiment, the liquid formulation may be for subcutaneous injection.

The liquid formulation may be formulated into a formulation suitable for the route of administration. For example, the liquid formulation may be formulated as an injection, an injectable ready-to-use form, and the like, but is not limited thereto.

The liquid formulation may be formulated such that the total amount or a pharmaceutically effective amount of an anti-IL-4Rα antibody (e.g., dupilumab) is provided in a single unit dosage form, or is divided across two or more (e.g., 2, 3, 4, 5, 6, 7, 8, 9, or 10) unit dosage forms. The liquid formulation may be included in a single-dose or multiple-dose form of a device.

The liquid formulation may be administered such that the total amount of the anti-IL-4Rα antibody (e.g., dupilumab) included in one unit dosage form is administered in vivo as a single bolus (e.g., within 1 minute, within 30 seconds, within 20 seconds, or within 10 seconds); or is administered as a gradual infusion in vivo over 5 minutes or more, 10 minutes or more, 30 minutes or more, 60 minutes or more, 90 minutes or more, 120 minutes or more, 150 minutes or more, 180 minutes or more, 210 minutes or more, or 240 minutes or more, but is not limited thereto.

A subject to be administered the liquid formulation may be selected from mammals including primates (e.g., humans, etc.), rodents (e.g., mice, rats, guinea pigs, hamsters, rabbits, etc.), cats, dogs, pigs, cows, horses, and the like.

The pharmaceutically effective amount of the liquid formulation or the anti-IL-4Rα antibody (e.g., dupilumab) included therein may mean an amount or dose sufficient to elicit a desired pharmacological effect, such as the elimination, mitigation, alleviation, or amelioration of symptoms. The pharmaceutically effective amount may be variously determined by factors such as a formulation method, a mode of administration, an age, a body weight, a sex, a pathological condition (severity of the condition) of a patient, a diet, an administration time, an administration interval, a route of administration, an excretion rate, a response sensitivity, a prior therapy, a clinical history, and the like. The dose may be adjusted at the discretion of an attending physician. The pharmaceutically effective amount may be administered at once, or divided and administered in multiple doses of two or more times.

For example, the liquid formulation may be administered once every 2 to 4 weeks over a period of 2 weeks or more at a dose such that the anti-IL-4Rα antibody (e.g., dupilumab) is 300 mg, 250 mg, 200 mg, 150 mg, or 100 mg.

The liquid formulation may be prepared as a general bulk formulation, and components of the liquid formulation are adjusted to a higher concentration than a concentration required for administration, and may be appropriately diluted and used prior to administration.

### Advantageous Effects of Invention

The liquid formulation of an anti-IL-4Rα antibody according to one aspect, despite being free of buffer, may have improved stability and improved viscosity compared to a buffer-containing formulation. Therefore, the liquid formulation maintains high stability and low viscosity even when containing the antibody at a high concentration, thereby enhancing patient convenience upon injection. Accordingly, the liquid formulation can be usefully utilized as a medicament for treating an IL-4Rα-related condition.

### Brief Description of Drawings

FIG. 1 is a graph showing the results of analyzing the high molecular weight species content (%HMW) for buffer-free formulations and buffer-containing formulations.
FIG. 2 is a graph showing the results of analyzing viscosity for buffer-free formulations and buffer-containing formulations.

### Best Mode for Carrying out the Invention

### Mode for the Invention

Hereinafter, the present disclosure will be described in more detail through the following examples. However, these examples are provided for illustrative purposes, and the scope of the present disclosure is not limited to these examples.

### [Experimental Methods]

### 1. Measurement of Viscosity

The viscosity of the sample was measured using a viscometer (manufacturer: RheoSense; model: VROC initium one plus). The analysis was conducted at a temperature of 25 °C during the viscosity measurement. Each sample was measured across 11 segments, and the viscosity of each sample was calculated by averaging the values having a slope fit R² of 0.9995 or greater.

### 2. Analysis of high molecular weight species content (%high molecular weight species, %HMW)

The percentage of high molecular weight species (%HMW) was determined using size exclusion chromatography (SEC) on a Waters HPLC system. The proteins were resolved into three distinct peaks based on molecular weight, corresponding to the HMW peak (referring to protein aggregation), the monomer peak, and the LMW peak (referring to protein degradation) in ascending order of retention time (i.e., in descending order of protein molecular weight). $- % HMW = \left\{area of HMW/area of \left(HMW+monomer+LMW\right)\right\} \times 100$

Each formulation sample for stability testing was stored in a chamber maintaining accelerated stability conditions at a temperature of 25±2 °C and a relative humidity (RH) of 60±5 %.

### [Examples]

### Example 1. Stability Test of Buffer-Free Formulations

An experiment was performed to confirm the effect of a buffer on the stability of a liquid formulation containing an anti-IL-4Rα antibody such as dupilumab.

Aqueous liquid formulations having the compositions shown in Table 1 below were prepared using dupilumab (CAS No. 1190264-60-8) as an anti-IL-4Rα antibody. After exposing each formulation to a temperature of 25 °C for 4 weeks, the purity of the samples was measured by size exclusion chromatography (SEC) analysis.

**[Table 1]**

| No. | Classification | Antibody Concentration | Buffer | pH | Excipient 1 | Excipient 2 | Surfactant | %HMW (25 °C, 4 weeks) |
|---|---|---|---|---|---|---|---|---|
| 1 | Buffer-free | | - | 5.9 | 5% (w/v) sucrose | 50 mM arginine | 0.2% (w/v) PS80 | 3.13 |
| 2 | | | | 5.9 | 5% (w/v) sucrose | 50 mM arginine | 0.2% (w/v) PS80 | 3.10 |
| 3 | | 175 mg/mL | | 5.9 | 5% (w/v) sucrose | 50 mM arginine | 0.2% (w/v) PS80 | 3.15 |
| 4 | Buffer-containing | | 10 mM sodium acetate | 6.0 | 4.3% (w/v) trehalose | 70 mM arginine | 0.2% (w/v) PS80 | 3.14 |
| 5 | | | | 6.0 | 1.8% (w/v) sorbitol | 70 mM arginine | 0.2% (w/v) PS80 | 3.21 |
| 6 | | | | 6.0 | 1.8% (w/v) mannitol | 70 mM arginine | 0.2% (w/v) PS80 | 3.38 |

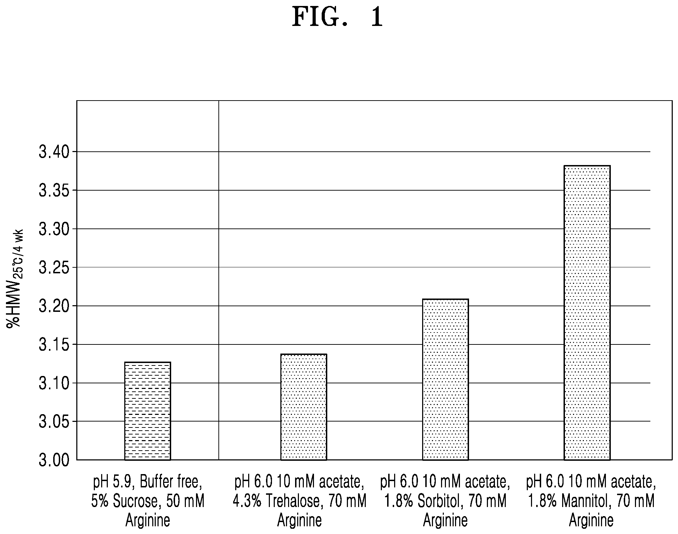

FIG. 1 is a graph showing the results of analyzing the high molecular weight species content (%HMW) for the buffer-free formulations and the buffer-containing formulations.

As a result, as shown in Table 1 and FIG. 1, the average %HMW value of the buffer-free formulations and the average %HMW value of the buffer-containing formulations were 3.13 % and 3.24 %, respectively.

Therefore, it was confirmed that the buffer-free formulations were more stable than the buffer-containing formulations.

### Example 2. Viscosity Test of Buffer-Free Formulations

An experiment was performed to confirm the effect of a buffer on the viscosity of a liquid formulation containing an anti-IL-4Rα antibody such as dupilumab.

The viscosity of the formulations prepared in Example 1 above was measured.

**[Table 2]**

| No. | Classification | Antibody Concentration | Buffer | pH | Excipient 1 | Excipient 2 | Surfactant | Viscosity (cP) |
|---|---|---|---|---|---|---|---|---|
| 1 | Buffer-free | | - | 5.9 | 5% (w/v) sucrose | 50 mM arginine | 0.2% (w/v) PS80 | 11.4 |
| 2 | | | | 5.9 | 5% (w/v) sucrose | 50 mM arginine | 0.2% (w/v) PS80 | 10.4 |
| 3 | | 175 mg/mL | | 5.9 | 5% (w/v) sucrose | 50 mM arginine | 0.2% (w/v) PS80 | 11.0 |
| 4 | Buffer-containing | | 10 mM sodium acetate | 6.0 | 4.3% (w/v) trehalose | 70 mM arginine | 0.2% (w/v) PS80 | 12.7 |
| 5 | | | | 6.0 | 1.8% (w/v) sorbitol | 70 mM arginine | 0.2% (w/v) PS80 | 11.6 |
| 6 | | | | 6.0 | 1.8% (w/v) mannitol | 70 mM arginine | 0.2% (w/v) PS80 | 14.2 |

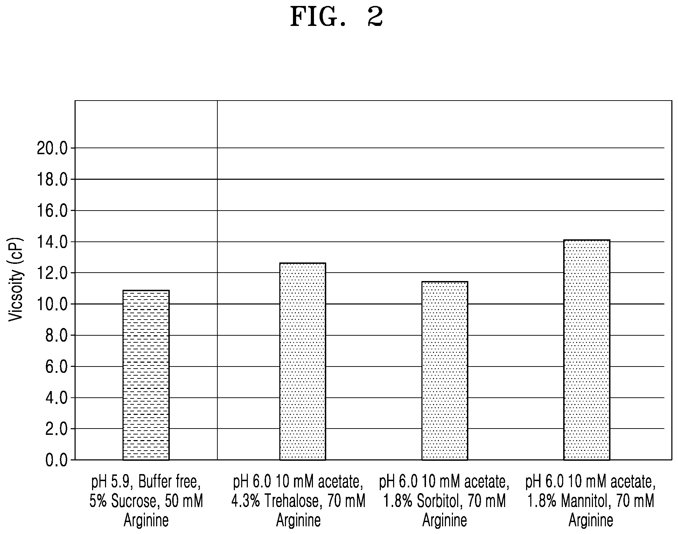

FIG. 2 is a graph showing the results of analyzing viscosity for the buffer-free formulations and the buffer-containing formulations.

As a result, as shown in Table 2 and FIG. 2, the average viscosity of the buffer-free formulations and the average viscosity of the buffer-containing formulations were 10.9 cP and 12.8 cP, respectively.

Therefore, it was confirmed that the buffer-free formulations had a lower viscosity than the buffer-containing formulations.

The above description of the present disclosure is provided for the purpose of illustration, and those of ordinary skill in the art to which the present disclosure pertains will understand that the present disclosure may be easily modified into other specific forms without changing the technical spirit or essential features thereof. Therefore, it should be understood that the embodiments described above are illustrative in all respects and not restrictive.

## Claims

1. A liquid formulation comprising:
an anti-IL-4Rα antibody;
a saccharide; and
an amino acid,
wherein the liquid formulation is free of buffer.

2. The liquid formulation of claim 1, wherein the anti-IL-4Rα antibody is dupilumab.

3. The liquid formulation of claim 1, wherein a concentration of the anti-IL-4Rα antibody is about 5 mg/mL to about 300 mg/mL.

4. The liquid formulation of claim 1, wherein a concentration of the anti-IL-4Rα antibody is about 100 mg/mL to about 200 mg/mL.

5. The liquid formulation of claim 1, wherein a concentration of the anti-IL-4Rα antibody is about 125 mg/mL to about 200 mg/mL.

6. The liquid formulation of any one of claims 1 to 5, wherein a pH of the liquid formulation is about 4.7 to about 7.4.

7. The liquid formulation of any one of claims 1 to 5, wherein a pH of the liquid formulation is about 5.5 to about 6.5.

8. The liquid formulation of claim 1, wherein the saccharide comprises one or more selected from a sugar and a sugar alcohol.

9. The liquid formulation of claim 8, wherein the sugar comprises one or more selected from sucrose, trehalose, galactose, mannose, maltose, lactose, fructose, and glucose.

10. The liquid formulation of claim 8, wherein the sugar alcohol comprises one or more selected from mannitol, sorbitol, xylitol, arabitol, erythritol, lactitol, maltitol, and inositol.

11. The liquid formulation of claim 8, wherein the saccharide is sucrose.

12. The liquid formulation of claim 8, wherein a concentration of the sugar is about 0.1% (w/v) to about 20% (w/v).

13. The liquid formulation of claim 8, wherein a concentration of the sugar alcohol is about 0.1% (w/v) to about 20% (w/v).

14. The liquid formulation of claim 11, wherein a concentration of the sucrose is about 2% (w/v) to about 10% (w/v).

15. The liquid formulation of claim 1, wherein the amino acid comprises one or more selected from glycine, alanine, valine, leucine, isoleucine, proline, phenylalanine, tyrosine, tryptophan, serine, threonine, cysteine, methionine, asparagine, glutamine, lysine, arginine, histidine, aspartic acid, and glutamic acid.

16. The liquid formulation of claim 1, wherein the amino acid comprises arginine.

17. The liquid formulation of claim 1, wherein a concentration of the amino acid is about 1 mM to about 400 mM.

18. The liquid formulation of claim 1, wherein a concentration of the amino acid is about 10 mM to about 100 mM.

19. The liquid formulation of any one of claims 1 to 18, further comprising a surfactant.

20. The liquid formulation of claim 19, wherein the surfactant comprises a nonionic surfactant.

21. The liquid formulation of claim 19, wherein the surfactant comprises polysorbate 20, polysorbate 80, or a combination thereof.

22. The liquid formulation of claim 19, wherein a concentration of the surfactant is 0.01% (w/v) to 0.9% (w/v).

23. The liquid formulation of any one of claims 1 to 22, wherein a high molecular weight species content (%HMW) of the antibody measured after storage at 25 °C for 4 weeks is 3.15 % or less.

24. The liquid formulation of any one of claims 1 to 22, wherein a viscosity of the liquid formulation is less than 11.5 cP.

25. The liquid formulation of any one of claims 1 to 24, wherein the liquid formulation is for subcutaneous injection.

26. A device comprising the liquid formulation of any one of claims 1 to 25.

27. The device of claim 26, wherein the liquid formulation is contained in a container selected from a syringe, a pre-filled syringe, a pre-filled pen, a microinfuser, an autoinjector, a bottle, a vial, and a tube.

28. A method of treating an IL-4Rα-related condition, comprising administering the liquid formulation of any one of claims 1 to 25 to a subject in need thereof.

29. The method of treating an IL-4Rα-related condition of claim 28, wherein the IL-4Rα-related condition is an IL-4-mediated disease or an IL-13-mediated disease.

30. The method of treating an IL-4Rα-related condition of claim 28, wherein the IL-4Rα-related condition is any one selected from atopic dermatitis, asthma, chronic rhinosinusitis (CRS), chronic rhinosinusitis with nasal polyposis (CRSwNP), allergic fungal rhinosinusitis (AFRS), eosinophilic esophagitis (EoE), prurigo nodularis (PN), bullous pemphigoid (BP), chronic spontaneous urticaria (CSU), chronic inducible urticaria (CIndU), and chronic obstructive pulmonary disease (COPD).

31. Use of a liquid formulation according to any one of claims 1 to 25 in the manufacture of a medicament for treating an IL-4Rα-related condition
